# EUROPEAN PATENT APPLICATION

(11) **EP 4 667 015 A1**
(43) Date of publication of application: **24.12.2025**
(21) Application number: 23922655.8
(22) Date of filing: 14.02.2023
(51) Int. Cl.: A61K 39/09, A01K 61/13, A61P 31/04, C12N 1/20

(54) **INACTIVATED VACCINE PREPARATION AND INFECTION PREVENTION METHOD**

(71) Applicant: Kyoritsu Seiyaku Corporation, Tokyo 102-0074 (JP)
(72) Inventor: UCHIYAMA Ai, Tsukuba-shi Ibaraki 300-1252 (JP); HANAOKA Naomi, Tsukuba-shi Ibaraki 300-1252 (JP); OKADA Hikaru, Tsukuba-shi Ibaraki 300-1252 (JP); MURAKAMI Taiga, Tsukuba-shi Ibaraki 300-1252 (JP); MIYADAI Hidenori, Tsukuba-shi Ibaraki 300-1252 (JP)
(74) Representative: Clark, Claudia
(86) International application number: PCT/JP2023/005050
(87) International publication number: WO 2024/171316

(57) **Abstract**

To isolate and identify a novel Lactococcus garvieae, and provide effective prevention means for diseases caused by the bacterium. Provided is an inactivated vaccine preparation against piscine streptococcosis caused by Lactococcus garvieae, comprising inactivated cells of Lactococcus garvieae that are negative for agglutination with anti-type I serum and anti-type II serum in a viable state. Occurrence, transmission, and spread of new type of streptococcosis that cannot be prevented by conventional inactivated vaccines against Lactococcus garvieae can be effectively prevented.

## Description

### [Technical Field]

The present invention relates to an inactivated vaccine preparation for piscine streptococcosis caused by a new serotype Lactococcus garvieae (scientific name: "Lactococcus garvieae", the same hereinafter), a method for preventing piscine streptococcosis, a method for producing an inactivated vaccine preparation, and the like. More specifically, the present invention relates to an inactivated vaccine preparation comprising inactivated cells of Lactococcus garvieae that are negative for agglutination with anti-type I serum and anti-type II serum in a viable state, a method for preventing piscine streptococcosis, a method for producing an inactivated vaccine preparation, and the like.

### [Background Art]

Piscine streptococcosis is a disease that occurs frequently, particularly in cultured fish, and causes significant economic losses. Piscine streptococcosis mainly includes α-hemolytic streptococcosis caused by Lactococcus garvieae and β-hemolytic streptococcosis caused by Streptococcus iniae (scientific name: "Streptococcus iniae").

Among these, piscine streptococcosis caused by Lactococcus garvieae largely occurs in Seriola such as yellowtail, amberjack and yellowtail amberjack, and also occurs in marine fish such as red sea bream, crimson porgy, and striped jack, and also in eels and rainbow trout, and the like. Symptoms in Seriola and the like include cloudy and protruding eyeballs, trunk deformation, redness inside the branchial mantle, pericarditis, and erratic swimming. Damage is large around the season when water temperatures is high.

Conventionally, when streptococcosis caused by Lactococcus garvieae occurred in aquaculture sites, the outbreak was calmed by carrying out fasting for a week or more, or by treatment with antibiotics such as erythromycin. However, in particular, for use of antibiotics, problems such as emergence of drug-resistant bacteria and concerns about residues of the antibiotics in food remain. Accordingly, in recent years, as a preventative measure against the disease, a vaccine has been developed and has already been released on the market.

Currently, examples of vaccine preparations for Lactococcus garvieae in fish include plain vaccine preparations, two-component combination vaccine preparations, and three-component combination vaccine preparations, including inactivated vaccines inactivated with formalin, concentrated inactivated vaccines from formalin-inactivated vaccines, inactivated vaccines obtained by enzymatically treating cultured bacterial liquid and then inactivating the liquid with formalin, and the like.

Conventionally, for Lactococcus garvieae, two serotypes, KG- type and KG+ type, were known. The KG- type is a strain that causes an agglutination reaction with anti-KG- serum but not cause an agglutination reaction with anti-KG+ serum, has a capsule, and is highly pathogenic. On the other hand, the KG+ type is a strain that causes an agglutination reaction with both anti-KG- and anti-KG+ serum, does not have a capsule, and is less pathogenic than the KG- type (see Non-Patent Documents 1 and 2).

However, in recent years, non-KG- type and non-KG+ type strains that do not agglutinate with diagnostic anti-KG- serum have been isolated from cultured fish (see Patent Document 1), causing a problem of expansion of infections in yellowtail fish aquaculture farms and the like. Currently, Lactococcus garvieae that agglutinates with conventional diagnostic antisera are classified as type I, and non-agglutinating strains are classified as type II (see Non-Patent Document 3). In other words, the type I refers to the previous KG- and KG+ strains, and the type II refers to the non-KG- and non-KG+ strains that have emerged in recent years. The type II is diagnosed not only by being non-agglutinating with conventional diagnostic antisera (anti-KG- serum) but also by being agglutinating with antisera produced from type II strains.

As fish vaccines related to Lactococcus garvieae, for example, Patent Document 1 discloses a vaccine preparation containing inactivated cells of non-KG- type/non-KG+ type Lactococcus garvieae, Patent Document 2 discloses a vaccine for piscine enterococcosis containing inactivated cells of a strain belonging to Lactococcus garvieae characterized by having a very thin capsule or no capsule, and Patent Document 3 discloses a vaccine for preventing and treating piscine streptococcosis using a novel strain. In addition, Non-Patent Document 4 discloses a method for distinguishing between type I and type II using the PCR method.

### [Citation List]

### [Patent Documents]

[Patent Document 1]
   Japanese Patent No. 6355512
[Patent Document 2]
   Japanese Patent Application Laid-Open No. H11-332558
[Patent Document 3]
   Japanese Patent Application Laid-Open No. 2001-103961
[Non-Patent Document 1]
   Yoshida, T., Eshima, T., Wada, Y., Yamada, Y., Kakizaki, E., Sakai, M., Kitao, T. and Inglis, V. (1996) "Phenotypic variation associated with an anti-phagocytic factor in the bacterial fish pathogen Enterococcus seriolicida." Dis Aquat Organ 25, 81-86.
[Non-Patent Document 2]
   M. Kawanishi, T. Yoshida, M. Kijima, K. Yagyu, T. Nakai, S. Okada, A. Endo, M. Murakami, S. Suzuki and H. Morita; "Characterization of Lactococcus garvieae isolated from radish and broccoli sprouts that exhibited a KG+ phenotype, lack of virulence and absence of a capsule"; Letters in Applied Microbiology 44 (2007) 481-487.
[Non-Patent Document 3]
   Yoshida, T., "Streptococcal infection and Lactococcus infection", Fish Pathology, 51(2), 44-48, 2016.6
[Non-Patent Document 4]
   Ohbayashi, K., Oinaka, D., Hoai, T.D., Yoshida, T. and Nishiki, I.; "PCR-mediated Identification of the Newly Emerging Pathogen Lactococcus garvieae Serotype II from Seriola quinqueradiata and S.dumerili; Fish Pathology, 52(1), 46-49, 2017.3

### [Summary of Invention]

### [Technical Problem]

The present invention aims to isolate and identify a novel Lactococcus garvieae and to provide effective prevention means for diseases caused by this bacterium.

### [Solution to Problem]

The present inventors originally isolated a new strain of Lactococcus garvieae from striped jack, amberjack, and yellowtail that were suspected of developing α-streptococcosis in spite of administration of a conventional inactivated vaccine against Lactococcus garvieae, in aquaculture sites in Shizuoka and Miyazaki prefectures, Japan, and found that the strain was different from the already known serotypes (types I and II). Then, the present inventors succeeded in identifying the strain as one that was negative for agglutination with anti-type I serum and anti-type II serum in a viable state, and succeeded in developing an inactivated vaccine against this new type of streptococcosis and demonstrating its effectiveness.

The present invention provides an inactivated vaccine preparation against piscine streptococcosis caused by Lactococcus garvieae, comprising an inactivated cell of Lactococcus garvieae that are negative for agglutination with anti-type I serum and anti-type II serum in a viable state, and related matters.

For example, administration of this inactivated vaccine preparation to fish may be able to effectively prevent occurrence, transmission, and spread of piscine streptococcosis caused by Lactococcus garvieae, the serotype of which is not classified as either known type I or type II, that is, a new type of streptococcosis that cannot be prevented by conventional inactivated vaccines against Lactococcus garvieae.

Furthermore, for example, by administering to fish a combined inactivated vaccine preparation comprising an inactivated vaccine comprising inactivated cells of Lactococcus garvieae that are negative for agglutination with anti-type I serum and anti-type II serum in a viable state, and an inactivated vaccine comprising inactivated cells of Lactococcus garvieae of serotype I (KG- type and/or KG+ type) and/or type II (non-KG- type and non-KG+ type), both the known streptococcosis and a new type of streptococcosis that is different from the known types may be able to be effectively prevented at the same time.

### [Advantageous Effect of the Invention]

The present invention can prevent occurrence, transmission, and spread of piscine streptococcosis caused by Lactococcus garvieae being negative for agglutination with anti-type I serum and anti-type II serum in a viable state.

### [Description of Embodiments]

### <Lactococcus garvieae LG21S and LG21M strains>

The present inventors originally isolated eight new strains of Lactococcus garvieae from striped jack or amberjack suspected of developing α-streptococcosis in spite of administration of a conventional inactivated vaccine against Lactococcus garvieae at aquaculture sites in Shizuoka and Miyazaki prefectures, Japan, and succeeded in identifying the strains as strains that were negative for agglutination with anti-type I serum and anti-type II serum in a viable state. Among the isolated and identified strains, the strain isolated from cultured striped jack in Shizuoka prefecture was named Lactococcus garvieae LG21S strain, and the strain isolated from cultured amberjack in Miyazaki prefecture was named Lactococcus garvieae LG21M strain, respectively.

The morphological characteristics of the LG21S and LG21M strains are identical to those of normal Lactococcus garvieae, and show the shape of an anaerobic gram-positive streptococcus. Motility and sporulation are not observed. As for the culture properties, white colonies are formed on commonly used meat extract agar plate media and casein-soybean mixed peptone agar plate media. Proliferation occurs by shaking culture on commonly used meat extract liquid media and casein-soybean mixed peptone liquid media. A culture temperature is suitably in a range of 20°C to 30°C.

The biochemical properties of the LG21S and LG21M strains are shown below.
(1) Gram staining: Gram positive
(2) Reduction of nitrates: -
(3) Denitrification reaction: -
(4) VP test: +
(5) Formation of indole: -
(6) Generation of hydrogen sulfide: -
(7) Use of citric acid: -
(8) Pigmentation: -
(9) Urease: -
(10) Oxidase: -
(11) Alanine-phenylalanyl-proline aryl amidase activity: +
(12) Pyroglutamate arylamidase activity: -
(13) N-acetyl-β-glucosaminidase activity: +
(14) Glycyl-tryptophan-arylamidase activity: -
(15) Growth range: pH 4.5 to 9.5, temperature 10 to 45°C
(16) Attitude towards oxygen: Facultative anaerobic
(17) Utilization of carbon sources: D-ribose: +, D-mannitol: +, D-sorbitol: -, lactose: -, D-trehalose: +, D-raffinose: -, saccharose: -, L-arabinose: -, D-arabitol: -, cyclodextrin: +, glycogen: -, pullulan: -, maltose: +, D-melibiose: -, melezitose: -, tagatose: +
(18) Decomposition of sugars: β-glucosidase activity: +, β-glucuronidase activity: +, β-galactosidase activity: -, α-galactosidase activity: -, β-mannosidase activity: -
(19) Decomposition of hippuric acid: -
(20) Decomposition of arginine: +
(21) Hemolysis: α-hemolytic
(22) Anti-KG- antiserum: -
(23) Anti-KG+ antiserum: -

Lactococcus garvieae LG21S and LG21M strains were deposited to patent microorganism deposit (depository institution: NITE Patent Microorganisms Depositary, Biological Resource Center, National Institute of Technology and Evaluation, Adress: 2-5-8 Kazusakamatari, Kisarazu-shi, Chiba, Japan, accession numbers: NITE BP-03560 and NITE BP-03561, date of receipt: November 19, 2021, strains collected in Japan).

Note here that the present invention is not limited to the use of the LG21S strain or LG21M strain, as long as it can effectively prevent new types of streptococcal infections caused by Lactococcus garvieae, which is negative for agglutination with anti-type I serum and anti-type II serum in a viable state, by inactivating the bacteria.

### <Lactococcus garvieae strain according to the present invention>

The present invention encompasses all strains of Lactococcus garvieae that are negative for agglutination with anti-type I serum and anti-type II serum in a viable state.

The cells of the present invention may be Lactococcus garvieae that is negative for agglutination with anti-type I serum and anti-type II serum in a viable state, that is, a new type of Lactococcus garvieae that is not classified as type I or type II. For example, the bacterial cells of the present invention may be isolated from fish that have developed α-streptococcosis in spite of administration of an existing inactivated vaccine against Lactococcus garvieae. The isolated bacteria can be cultured and proliferated in known solid or liquid media, such as meat extract agar plate medium, casein-soybean mixed peptone agar plate medium, meat extract liquid medium, or casein-soybean mixed peptone liquid medium. Note here that in the present invention, the term "viable state" refers to a state in which bacteria are alive, or a state in which the three-dimensional shape of the bacterial antigen is maintained in the same manner as in the case where the bacteria are alive.

The Lactococcus garvieae of the present invention includes those having the serological characteristics of being negative for agglutination with anti-type I serum and anti-type II serum in the viable state, as well as being positive for agglutination with anti-type I serum in heat-killed cells. The fact that agglutination with anti-type I serum is positive after heat treatment, although agglutination with anti-type I serum is negative in a viable state, suggests that due to a mutation, the recognition site of the anti-type I serum in the viable state is covered with some kind of capsule-like structure, and that the structure is removed by heat treatment, allowing anti-type I serum to reach the recognition site. The reason why existing vaccines against type I or type II Lactococcus garvieae are not effective against novel streptococcosis can be supported by the above mention.

In addition to the above, the Lactococcus garvieae of the present invention also includes those having the serological characteristic that the heat-killed cells are positive for agglutination with anti-KG- type serum and anti-KG+ type serum. This case suggests that the new type streptococcosis may be caused by a mutant strain of the type I/KG+ type Lactococcus garvieae.

The Lactococcus garvieae of the present invention broadly includes those including the sequence set forth in SEQ ID NO: 1 in the glxR coding region or in the vicinity thereof in the genome. In the sequence set forth in SEQ ID NO: 1, three base mutations are observed compared to the base sequences of the existing type I or type II Lactococcus garvieae (a mutation from adenine to cytosine at position 62 in the sequence of SEQ ID NO: 1, a deletion of positions 118 to 119, and a deletion of positions 141 to 142). These mutations may contribute to the change in the serological characteristics of the new type of Lactococcus garvieae compared to the conventional ones.

The LG21S strain or the LG21M strain are suitable as the Ctococcus garvieae cells used in the present invention because they can be used as a vaccine after inactivation, and because they are highly effective as a vaccine even without concentration or enzyme treatment.

### <Inactivated vaccine preparation according to the present invention>

The present invention includes all inactivated vaccine preparations against piscine streptococcosis caused by Lactococcus garvieae, comprising any of the above-mentioned inactivated cells of Lactococcus garvieae. Furthermore, the present invention is not narrowly limited to those comprising inactivated cells as an active component, but also broadly includes inactivated vaccine preparations comprising, as active components, cells and a bacterial liquid obtained by inactivating a cultured bacterial liquid of Lactococcus garvieae that is negative for agglutination with anti-type I serum and anti-type II serum in a viable state, that is, for example, those comprising inactivated cells and cell-derived components other than the cells by using the inactivated cells and the culture liquid without isolating them, or by concentrating the inactivated cells and the culture liquid without isolating them.

An inactivated cell obtained by inactivating any of the above-mentioned new Lactococcus garvieae strains, for example, a Lactococcus garvieae strain that is negative for agglutination with anti-type I serum and anti-type II serum in a viable state, is useful as an inactivated vaccine preparation for preventing new piscine streptococcosis.

As the inactivated cells, for example, any of the above-mentioned new Lactococcus garvieae strains can be cultured and proliferated, and the cultured bacterial liquid can be inactivated.

Inactivation of cells can be carried out, for example, by subjecting the cultured bacterial liquid to physical treatment (ultraviolet light irradiation, X-ray irradiation, heat treatment, ultrasonic treatment, and the like) or chemical treatment (treatment with organic solvents such as formalin or chloroform, acid treatment with weak acids such as acetic acid, treatment with alcohol, chlorine, mercury, and the like).

For example, formalin can be added to the cultured bacterial liquid at a volume concentration of 0.001 to 2.0%, and more preferably 0.01 to 1.0%, and the cultured bacterial liquid is then sensitized at 4 to 30°C for 1 to 10 days to perform inactivation with formalin. For example, the inactivated cells may be washed with a buffer solution or the like to remove the inactivating agent such as formalin, or the inactivated cells may be neutralized by adding a neutralizing agent. In addition, the inactivated cells may be collected by membrane filtration or centrifugation, or the cultured liquid may be concentrated without isolating the cells from the culture solution.

The amount of inactivated cells included in the inactivated vaccine preparation is not particularly limited, but, for example, the amount of cells before inactivation is preferably in the range of 10³ to 10¹¹ CFU/mL, and more preferably in the range of 10⁷ to 10¹¹ CFU/mL.

The inactivated vaccine preparation of the present invention may comprise an adjuvant, that is, may comprise at least the above-mentioned inactivated cells and an adjuvant as active components.

A wide variety of known adjuvants can be used. Examples of the adjuvant include oil-based adjuvants including animal oils (such as squalene) or hydrogenated oils thereof, vegetable oils (such as palm oil and castor oil) or hydrogenated oils thereof, anhydrous mannitol oleic ester, liquid paraffin, polybutene, caprylic acid, oleic acid, and higher fatty acid esters; water-soluble adjuvants such as PCPP, saponin, manganese gluconate, calcium gluconate, manganese glycerophosphate, soluble aluminum acetate, aluminum salicylate, acrylic acid copolymer, methacrylic acid copolymer, maleic anhydride copolymer, alkenyl derivative polymer, oil-in-water emulsion, and cationic lipids comprising quaternary ammonium salts; precipitating adjuvants such as aluminum hydroxide (alum) and sodium hydroxide; microbial toxin components such as cholera toxin and E. coli heat-labile toxin; and bentonite, muramyl dipeptide derivatives, interleukins, and the like. Furthermore, a mixture thereof may be used.

The present invention may be a combined inactivated vaccine preparation comprising the above-mentioned inactivated vaccine, for example, an inactivated vaccine comprising inactivated cells of Lactococcus garvieae that are negative for agglutination with anti-type I serum and anti-type II serum in a viable state, and an inactivated vaccine comprising inactivated cells of Lactococcus garvieae type I (KG-type and/or KG+ type) and/or type II (non-KG- type and non-KG+ type). By administering this combined inactivated vaccine preparation to fish, both the known streptococcosis and a new type of streptococcosis that is different from the known streptococcosis can be effectively prevented at the same time.

In addition to an inactivated vaccine comprising inactivated cells of Lactococcus garvieae that are negative for agglutination with anti-type I serum and anti-type II serum in a viable state, or a combined inactivated vaccine comprising the inactivated vaccine and inactivated cells of Lactococcus garvieae of type I (KG- type and/or KG+ type) and/or type II (non-KG- type and non-KG+ type), the vaccine may also be a combined vaccine preparation further including any one or more of vaccines against other diseases, such as a β-hemolytic streptococcosis inactivated vaccine, a vibriosis inactivated vaccine, an iridovirus disease inactivated vaccine, a pseudotuberculosis inactivated vaccine, and a Streptococcus dysgalactiae infection inactivated vaccine.

In addition, a buffer agent, an isotonicity agent, a soothing agent, a preservative, an antibacterial agent, or an antioxidant may be added appropriately depending on the purpose and use.

Suitable examples of the buffer agent include buffer solutions such as phosphate, acetate, carbonate, and citrate.

Suitable example of the isotonicity agent include sodium chloride, glycerin, and D-mannitol.

Suitable example of the soothing agent includes benzyl alcohol.

Suitable examples of the agent for preservative purposes include thimerosal, paraoxybenzoic acid esters, phenoxyethanol, chlorobutanol, benzyl alcohol, phenethyl alcohol, dehydroacetic acid, sorbic acid, and various other preservatives, antibiotics, and synthetic antibacterial agents.

Suitable examples of the antioxidant include sulfites and ascorbic acid.

In addition, this drug may comprise auxiliary components such as light-absorbing pigments (riboflavin, adenine, adenosine, and the like) that aid in preservation and efficacy, chelating agents and reducing agents (vitamin C, citric acid, and the like) for stabilization, carbohydrates (sorbitol, lactose, mannitol, starch, sucrose, glucose, dextran, and the like), casein digests, various vitamins, and the like.

The dosage form of the vaccine preparation is not particularly limited, and can be any known form. For example, a liquid preparation may be used, or the preparation may be mixed into feed or the like after processing such as freeze-drying.

### <Method for producing inactivated vaccine preparation according to the present invention>

The present invention encompasses all methods for producing inactivated vaccine preparations against piscine streptococcosis caused by Lactococcus garvieae, the method comprising inactivating any of the above-mentioned Lactococcus garvieae cells.

As described above, for example, by inactivating Lactococcus garvieae cells that are negative for agglutination with anti-type I serum and anti-type II serum in a viable state, it is possible to produce an inactivated vaccine preparation that is effective against a new type of piscine streptococcosis caused by Lactococcus garvieae that is negative for agglutination with anti-type I serum and anti-type II serum in a viable state.

The inactivated vaccine preparation of the present invention can be produced, for example, by proliferation of Lactococcus garvieae cells that are negative for agglutination with anti-type I serum and anti-type II serum in a viable state, and by inactivating the cells. After the inactivation process, a process of adding an adjuvant or the like to the inactivated cells may be added appropriately.

The cells used and the method for inactivating the cells are as described above. Furthermore, the above-mentioned adjuvant, a buffer agent, an isotonicity agent, a soothing agent, a preservative, an antioxidant, or the like, may be added appropriately depending on the purpose and use.

### <Use of bacteria for production of inactivated vaccine preparations>

The present invention broadly encompasses the use of the bacteria for producing an inactivated vaccine preparation against streptococcosis caused by any of the above Lactococcus garvieae.

For example, in order to produce an inactivated vaccine preparation for streptococcosis caused by Lactococcus garvieae, which is negative for agglutination with anti-type I serum and anti-type II serum in a viable state, the above-mentioned Lactococcus garvieae, which is negative for agglutination with anti-type I serum and anti-type II serum in a viable state, can be widely used.

### <Method for preventing piscine streptococcosis according to the present invention>

Methods for preventing piscine streptococcosis caused by Lactococcus garvieae, comprising administering the inactivated vaccine preparation or the combined inactivated vaccine preparation described above are widely included.

Administration of the above-mentioned inactivated vaccine preparation to fish can prevent occurrence, transmission, and spread of new types of piscine streptococcosis that cannot be prevented by conventional inactivated vaccines. Furthermore, administration of the above-mentioned combined inactivated vaccine preparation to fish may be able to effectively prevent both the known streptococcosis and new types of streptococcosis that are different from the known streptococcosis at the same time.

Examples of the subject fish include fish of the genus Seriola (yellowtail, amberjack, yellowtail amberjack, and the like), sea fish such as red sea bream, red sea bream, flounder, striped jack, horse mackerel, mackerel, and tuna, as well as eels and rainbow trout, which can be infected with streptococcosis caused by Lactococcus garvieae.

Examples of a method for administering the inactivated vaccine preparation include injection, immersion, and oral administration.

In the case of the injection method, for example, 0.05 to 3.0 mL of an inactivated vaccine preparation, in which the amount of cells before inactivation is adjusted to be in the range of 10³ to 10¹¹ CFU/mL, is administered intramuscularly or intraperitoneally. In other words, an inactivated vaccine preparation in which the amount of cells before inactivation is 10³ to 10¹¹ CFU/mL and the amount administered per dose is 0.05 to 3.0 mL, and this dose of an inactivated vaccine preparation administered intramuscularly or intraperitoneally, is effective in preventing new types of piscine streptococcosis that cannot be prevented by conventional inactivated vaccines against Lactococcus garvieae.

In the case of the immersion method, for example, subject fish are immersed for 0.05 to 48 hours in a liquid comprising an inactivated vaccine preparation in which the amount of cells before inactivation is adjusted to be in the range of 10³ to 10¹¹ CFU/mL. In other words, an inactivated vaccine preparation, which has an amount of cells before inactivation being 10³ to 10¹¹ CFU/mL and is used for immersion for 0.05 to 48 hours per dose, is effective in preventing a new type of piscine streptococcosis that cannot be prevented by conventional inactivated vaccines against Lactococcus garvieae.

In the case of the oral method, for example, subject fish are fed ad libitum with a feed including an inactivated vaccine formulation prepared to have an amount of cells before inactivation in the range of 10³ to 10¹¹ CFU/mL, and continuously administered for 1 to 20 days. In other words, an inactivated vaccine preparation in which the amount of cells before inactivation is 10³ to 10¹¹ CFU/mL, and which is administered orally for 1 to 20 days is effective in preventing new types of streptococcosis that cannot be prevented by conventional inactivated vaccines against Lactococcus garvieae.

Among these, intraperitoneal administration by injection is the most suitable because it is highly effective in preventing infection and provides long-lasting immunity.

The inactivated vaccine preparation may be administered once as long as its effect lasts, but may also be administered multiple times, for example at intervals of 7 to 365 days, depending on the size of subject fish and the degree of vaccine effectiveness. In addition, the inactivated vaccine preparation may be administered to the subject fish by appropriately combining multiple administration methods.

### [Example 1]

In Example 1, isolation and identification of the causative bacteria were attempted for striped jack and amberjack suspected of developing α-streptococcosis in spite of administration of a conventional combined inactivated vaccine against Lactococcus garvieae types I and II.

Since around July 2021, in a striped jack aquaculture farm in Shizuoka Prefecture, Japan, and a striped jack and amberjack aquaculture farm in Miyazaki Prefecture, Japan, several individuals suspected of α-hemolytic streptococcosis were found in cultured fish groups to which the conventional mixed inactivated vaccine against L had been administered. Six individuals of them were caught and used as specimens.

Significant findings from the autopsy of each specimen in visual examination included opacity or redness of the eyeball, redness around the eyeball, redness and ulcers at the base of the caudal fin, defects of the dorsal and pectoral fins, redness of the pectoral, ventral, and gluteal fins. Internal findings showed redness of the inner operculum, discoloration of the gills, pericarditis, and enlargement of the spleen.

The kidney, spleen, or brain of the specimen was punctured, streaked onto SCDb agar medium (a casein-soybean mixed peptone liquid medium to which agar had been added), and cultured at 25°C for 24 hours. Single colonies were then picked and inoculated onto new SCDb agar medium for single colony isolation. By this procedure, a total of eight strains of bacteria that form white S-type colonies were isolated: six from cultured striped jack in Shizuoka Prefecture, one from cultured striped jack in Miyazaki Prefecture, and one from cultured amberjack in Miyazaki Prefecture.

Microscopic examination of the isolated bacteria revealed that all isolated bacteria had the morphology of a gram-positive streptococcus, similar to known L. garvieae.

A hemolytic test was carried out on the isolated bacteria. The isolated bacteria were inoculated onto Columbia 5% sheep blood agar medium and cultured. As a result, incomplete hemolytic rings similar to those of known L. garvieae were formed in all isolated strains, showing α-hemolysis.

The isolated strains were subjected to slide agglutination tests still in a viable state. Among the isolated strains, one strain isolated from cultured striped jack in Shizuoka Prefecture, one strain isolated from cultured amberjack in Miyazaki Prefecture, and five strains, type I/KG- type (KS-7M strain), type I/KG+ type (YT-3 strain), and type II (LG13E strain) as comparative examples, were picked from colonies and suspended in diagnostic rabbit antisera. Three types of diagnostic rabbit antisera were used: anti-KG- type serum and anti-KG+ type serum as anti-type I serum, and anti-type II serum (anti-non-KG-/KG+ serum). The results are shown in Table 1. As shown in Table 1, both the isolated strains were negative for agglutination with all antisera.

**[Table 1]**

| | | | **Anti-type I serum** | | **Anti-type II serum** |
|---|---|---|---|---|---|
| | | | **Anti-KG-serum** | **Anti-KG+ serum** | **Anti-non KG-/KG+ serum** |
| **Type I** | **KG-** | **KS-7M** | + | - | - |
| | **KG+** | **YT-3** | + | + | - |
| **Type II** | **Non-KG-/KG+** | **LG13E** | - | - | + |
| **Isolated strain** | | ① | - | - | - |
| | | ② | - | - | - |

Next, a slide agglutination test was carried out on the heat-killed cells of the isolated strains. Among the isolated strains, one of the strains isolated from the cultured striped jack in Shizuoka Prefecture, one isolated from cultured amberjack in Miyazaki Prefecture, and four strains, type I/KG- type (KS-7M strain) and type I/KG+ type (YT-3 strain) as comparative examples, were picked from colonies, suspended, heated at 100°C for 5 minutes, and then suspended in diagnose rabbit antiserum. For the diagnostic rabbit antiserum, three types of anti-type I serum were used: anti-KG- type serum, anti-KG+ type serum, and anti-type II serum (anti-non-KG-/KG+ serum). The results are shown in Table 2. As shown in Table 2, unlike the case where the isolated strains were agglutinated in a viable state, when the anti-type I serum was used, both the isolated strains were positive for agglutination with both the anti-KG- type serum and the anti-KG+ type serum. When the anti-type II serum (anti-non-KG-/KG+ serum) was used, the isolated strains were negative for agglutination.

**[Table 2]**

| | | | Anti-type I serum | | Anti-type II serum |
|---|---|---|---|---|---|
| | | | Anti-KG-serum | Anti-KG+ serum | Anti-non KG-/KG+ serum |
| I type | KG- | KS-7M | + | - | - |
| | KG+ | YT-3 | + | + | - |
| Isolated strain | | ① | + | + | - |
| | | ② | + | + | - |

Genetic test of the isolated strains was carried out. Using primers for distinguishing L. garvieae type I/II described in Non-Patent Document 4, detection of genes specific to type I or type II by PCR was attempted. As a result, amplification of the genes specific to L. garvieae type I was observed in all isolated strains, but amplification of genes specific to type II was not observed.

The results of the drug susceptibility test showed that the bacteria isolated from the brain formed inhibition circle on erythromycin, doxycycline, thiamphenicol, florfenicol, and oxytetracycline.

Viral test was performed. DNA was extracted from the spleen of the specimens, and red sea bream iridovirus (RSIV)-specific genes were attempted to be detected using real-time PCR. No amplification of RSIV-specific genes was observed in any of the specimens.

An acid-fast bacterium test was carried out. The kidney of each specimen was punctured, streaked onto 1% Ogawa medium, and cultured at 28°C for 7 days in an attempt to isolate bacterium. As a result, no growth of the acid-fast bacterium was observed on the 1% Ogawa medium.

The biochemical properties of the isolated bacteria were tested. Using the Gram-positive cocci identification kit "rapid ID32 STREP (Nippon BioMérieux)," tests were carried out on three strains in total: two strains the same as those used in the slide agglutination test, and one strain isolated from aquaculture striped jack in Miyazaki Prefecture. The results are shown in Tables 3 and 4. The results for the three strains tested were all the same. In addition, in Tables 3 and 4, "positive rate" refers to the positive rate of known L. garvieae as stated in the kit's package insert.

**[Table 3]**

| **Item** | **Reaction substrate** | **Isolated bacteria** | **Positive rate** |
|---|---|---|---|
| ADH | L-arginine | + | 100% |
| βGLU | p-nitrophenyl-β-D-glucopyranoside | + | 100% |
| βGAR | p-nitrophenyl-β-D-galactopyranoside | - | 0% |
| βGUR | p-nitrophenyl-β-D-glucuronide | - | 0% |
| αGAL | p-nitrophenyl-α-D-galactopyranoside | - | 0% |
| PAL | p-nitrophenyl phosphate bis(cyclohexylammonium) salt | - | 0% |
| RIB | D-ribose | + | 35% |
| MAN | D-mannitol | + | 75% |
| SOR | D-sorbitol | - | 0% |
| LAC | Lactose | - | 50% |
| TRE | D-trehalose | + | 100% |
| RAF | D-raffinose | - | 0% |
| SAC | Saccharose | - | 50% |
| LARA | L-arabinose | - | 0% |
| DARL | D-arabitol | - | 0% |
| CDEX | Cyclodextrin | + | 50% |

**[Table 4]**

| **Item** | **Reaction substrate** | **Isolated bacteria** | **Positive rate** |
|---|---|---|---|
| VP | Sodium pyruvate | + | 100% |
| APPA | L-alanyl-L-phenylalanyl-L-proline-β-naphthylamide | + | 100% |
| βGAL | 2-naphthyl-β-D-galactopyranoside | - | 0% |
| PyrA | L-pyroglutamic acid-β-naphthylamide | - | 74% |
| βNAG | 6 bromo-2 naphthyl-N-acetyl-β-D-glucosaminide | + | 10% |
| GTA | Glycyl-L-tryptophan-β-nartylamide | - | 0% |
| HIP | Sodium hippurate | - | 0% |
| GLYG | Glycogen | - | 0% |
| PUL | Pullulan | - | 0% |
| MAL | Maltose | + | 75% |
| MEL | D-melibiose | - | 0% |
| MLZ | Meletitose | - | 0% |
| MβDG | Methyl-β-D-glucopyranoside | + | 85% |
| TAG | Tagatose | + | 50% |
| βMAN | 4-Nitrophenyl-β-D-mannopyranoside | - | 0% |
| URE | Urea | - | 0% |

As a comparative example, the same biochemical property tests were also carried out on the L. garvieae type I/KG-type (KS-7M strain) and the type II (LG13E strain), and the results were compared. In comparison with the L. garvieae type I/KG-type (KS-7M strain), the strain was negative in the fermentation/oxidation ability of D-ribose and tagatose, whereas all the isolated strains were positive. In comparison with the L. garvieae type II (LG13E strain), the strain was negative in the βNAG item, whereas all the isolated strains were positive. It was difficult to distinguish positive/negative for the lactose fermentation/oxidation ability item for the isolated strains. All other items were the same for a total of five strains: the L. garvieae type I/KG- type (KS-7M strain), the type II (LG13E strain), and the three isolated strains.

As described above, as new types of strain being negative for agglutination with both anti-L. garvieae type I serum (anti-KG-type serum and anti-KG+ type serum) and anti-L. garvieae type II serum in a viable state, but being positive for agglutination with the anti-L. garvieae type I serum in heat-killed cells, that is, having different serological characteristics from the conventional types I and II, the eight strains in total were successfully isolated and identified, including six from aquaculture amberjack in Shizuoka Prefecture, one from aquaculture amberjack in Miyazaki Prefecture, and one from aquaculture amberjack in Miyazaki Prefecture. One of the strains isolated from the cultured striped jack in Shizuoka Prefecture was named LG21S strain and deposited at NITE Patent Microorganisms Depositary, Biological Resource Center of National Institute of Technology and Evaluation (Accession Number: NITE BP-03560). Furthermore, one strain isolated from cultured amberjack in Miyazaki Prefecture was named LG21M strain and deposited at NITE Patent Microorganisms Depositary, Biological Resource Center of National Institute of Technology and Evaluation (Accession Number: NITE BP-03561).

### [Example 2]

In Example 2, pathogenicity of the strain isolated in Example 1 was checked.

The test fish were prepared in groups of 60 each of artificial yellowtail (delivered in February 2021, average body weight: about 25 g), artificial amberjack (delivered in February 2021, average body weight: about 28 g), and artificial striped jack (delivered in February 2021, average body weight: about 29 g).

Among the strains isolated in Example 1, the LG21S strain and another strain (LC2115 strain) isolated from an aquaculture striped jack in Shizuoka Prefecture were frozen and stored at -80°C, and each isolated strain was streaked on SCDb agar medium and cultured at 25°C for about 24 hours. The grown colonies were suspended in PBS, and 1 mL of the suspension was prepared to approximately McFaland No. 3-4, and inoculated into 9 mL of SCDb liquid medium, and similarly diluted 10-fold in stages until 10⁻¹⁰-fold. After static culture for about 24 hours at 25°C, the one that was one step before the dilution step that reached the peak in turbidity was selected and diluted in three stages of 10, 100, and 1,000-fold, with PBS were used as test bacterial solutions.

The artificial yellowtail, artificial amberjack, and artificial striped jack were divided into six groups of 10 fish each, with three groups each for inoculation with the LG21S strain and three groups for inoculation with the LC2115 strain. Under anesthesia, 0.1 mL of bacterial liquid prepared in three levels of 10-fold concentration was injected intraperitoneally per fish, and the fish were reared at 25°C for 14 days after injection, and the mortality rates were compared.

As a result, the cumulative mortality rates up to 14 days after the injection of the LG21S strain (1.3 x 10⁵ to 10⁷ CFU per fish) were 50, 80, and 90% in yellowtail, 100, and 90 and 100% in amberjack and striped jack, respectively, in the order from the lower concentration. The cumulative mortality rates up to 14 days after the injection of the LC2115 strain (2.3 x 10⁵ to 10⁷ CFU per fish) were 40, 50, and 100% in yellowtail, 90, 100, and 100% in amberjack, and 100% in striped jack, respectively, in the order from the lower concentration.

In surviving amberjacks after inoculation with each bacterial solution, lesions characteristic of alpha-hemolytic streptococcosis were observed.

Bacteria were reisolated from the organs of dead fish, and the characteristics the same as those of the strains used in infection (LG21S strain or LC2115 strain) were observed. Furthermore, slide agglutination tests were carried out on the reisolated bacteria, and agglutination was positive when the anti-type I serum was used only after heat treatment, as in the strains used in infection (LG21S strain or LC2115 strain).

As described above, this example shows that the strain isolated in Example 1 is highly pathogenic in each fish species and causes pathological conditions similar to those observed in each fish aquaculture farm.

### [Example 3]

In Example 3, an inactivated vaccine against α-streptococcosis caused by a new serotype of L. garvieae was produced, which were negative for agglutination with both anti-L. garvieae type I serum (anti-KG- type serum and anti-KG+ type serum) and anti-L. garvieae type II serum in a viable state, but were positive for agglutination with the anti-L. garvieae type I serum in heat-killed cells, and its efficacy as a vaccine was verified.

The inactivated bacterial solution was prepared as follows. The new serotype LG21S strain, type I and KG- type (KS-7M strain), and type II (LG13E strain) were used as test strains. Each test strain, frozen and stored at -80°C, was streaked on an SCDb agar medium and cultured at 25°C for about 24 hours. The grown colonies were suspended in PBS, and 100 µL of the suspension was prepared to approximately McFaland No. 3-4, inoculated into 100 mL of SCDb liquid medium and cultured at 25°C for about 24 hours with shaking at about 90 rpm. Formalin was added to this cultured bacterial liquid so that the concentration became 0.5 vol%, and the mixture was shaken for about 48 hours to sensitize the bacteria, thereby an inactivated bacterial solution was prepared. Each inactivated bacterial solution was centrifuged at 4°C and 12,000 G for 10 minutes to remove the supernatant, and then formalin was added thereto so that the concentration became 0.2 vol% and resuspended in PBS, and the antigen amount was adjusted to be equal between the strains, thereby the test antigen was prepared. The amount of antigen was adjusted to 1.6 x 10⁹ CFU/mL in terms of viable bacterial count before inactivation.

As the test fish, 144 artificial yellowtails (delivered in June 2021, average body weight: about 25 g) and 144 artificial amberjacks (delivered in June 2021, average body weight: about 28 g) were prepared.

The artificial yellowtails and artificial amberjacks were divided into six groups of 18 fish each, and each group was injected intraperitoneally with 0.1 mL of the test antigen per fish under anesthesia, creating groups immunized with the LG21S strain, KS-7M strain, and LG13E strain, respectively. After injection, the fish were reared at 25°C for 14 days. Note here that the control group was similarly injected with PBS.

The LG21S strain was selected as the challenge strain and prepared for challenge. The strain, which had been frozen and stored at -80°C, was streaked on SCDb agar medium and cultured at 25°C for about 24 hours. The grown colonies were suspended in PBS, and 1 mL of this was prepared to approximately McFaland No. 3-4, inoculated into 9 mL of SCDb liquid medium, and similarly diluted 10-fold to 10⁻¹⁰-fold. Static culture was carried out for about 24 hours at 25°C, and the one that had reached the peak in turbidity was selected, and diluted 100-fold or 1,000-fold with PBS. The resultant bacterial solution was used as the challenge bacterial solution.

The fish were challenged by intraperitoneally injecting 0.1 mL of the challenge solution per fish under anesthesia 14 days after immunization. After the challenge, the fish were reared in a water tank at a set temperature of 25°C for 14 days, and the survival rates were compared.

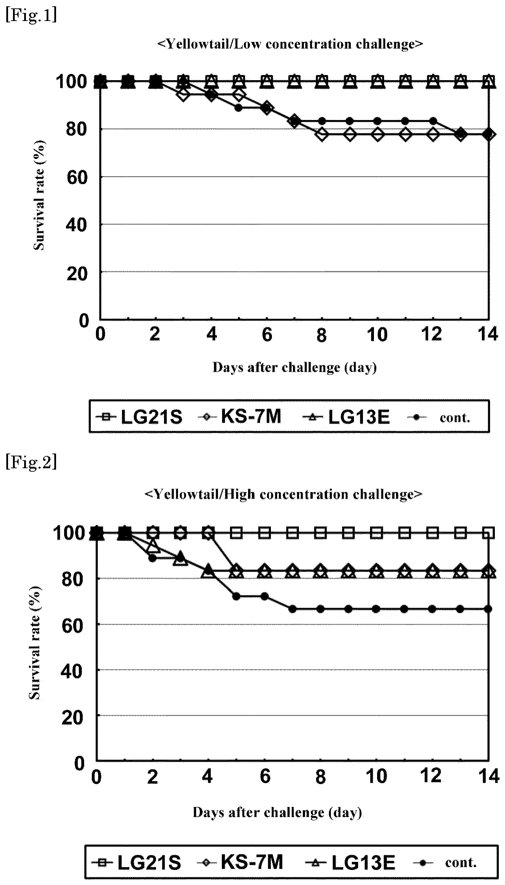

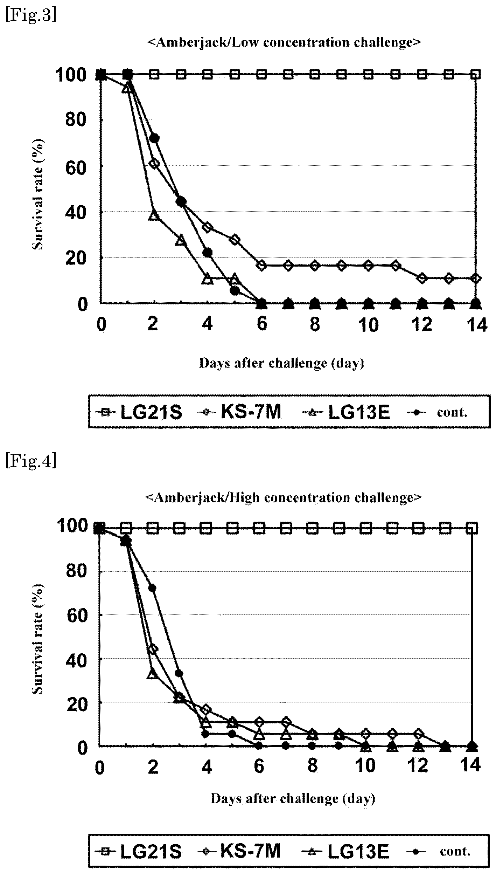

The results are shown in FIGs. 1 to 4. FIG. 1 is a graph showing the survival rate of artificial yellowtails immunized with inactivated cells of the new serotype L. garvieae LG21S strain and then challenged with the LG21S strain at a low concentration (8.0 x 10⁴ CFU/0.1 mL per fish); FIG. 2 is a graph showing the survival rate of artificial yellowtails immunized with inactivated cells of the new serotype L. garvieae LG21S strain and then challenged with the LG21S strain at a high concentration (8.0 x 10⁵ CFU/0.1 mL per fish); FIG. 3 is a graph showing the survival rate of artificial amberjack immunized with inactivated cells of the new serotype L. garvieae LG21S strain and then challenged with the LG21S strain at a low concentration (8.0 x 10⁴ CFU/0.1 mL per fish); and FIG. 4 is a graph showing the survival rate of artificial amberjack immunized with inactivated cells of the new serotype L. garvieae LG21S strain and then challenged with the LG21S strain at a high concentration (8.0 x 10⁵ CFU/0.1 mL per fish), respectively. In each graph, the horizontal axis (days after challenge) of the graph represents the days after the challenge, and the vertical axis (survival rate, unit: %) represents the survival rate after the challenge. In the graph, the line of "LG21 S" represents the survival rate when immunization with inactivated cells of the new serotype L. garvieae LG21S strain was carried out, the line of "KS-7M" represents the survival rate when immunization with inactivated cells of L. garvieae type I and KG- type (KS-7M strain) was carried out as a comparative example, the line of "LG13E" represents the survival rate when immunization with inactivated cells of L. garvieae type II (LG13E strain) was carried out as a comparative example, and the line of "control group" represents the survival rate when PBS was administered as a control.

When artificial yellowtails were immunized, as shown in FIG. 1, the survival rate 14 days after low-concentration challenge with the LG21S strain (8.0 x 10⁴ CFU/0.1 mL per fish, intraperitoneal injection) was 78% in the KS-7M strain-immunized group, and 100% in the LG13E strain-immunized group, and 100% in the LG21S strain-immunized group, as compared with 78% in the control group, showing no significant difference between the control group and each immunization group. However, among the surviving fish 14 days after challenge, many individuals in the control, KS-7M strain-immunized, and LG13E strain-immunized groups were found to have developed α-hemolytic streptococcosis, although they did not die. When these were subtracted, the LC2113 strain-immunized group showed a significantly higher normal rate than the control group. Furthermore, as shown in FIG. 2, the survival rate 14 days after high-concentration challenge (8.0 x 10⁵ CFU/0.1 mL per fish, intraperitoneal injection) of artificial yellowtail with the LG21S S strain was 83% in the KS-7M strain immunized group, 83% in the LG13E strain immunized group, and 100% in the LG21S strain immunized group, as compared with 67% in the control group, showing that the LG21S strain immunized group had a significantly higher survival rate than the control group (p < 0.05, Fisher's exact test, one-sided test).

When artificial amberjacks were immunized, as shown in FIG. 3, the survival rate 14 days after low-concentration challenge (8.0 x 10⁴ CFU/0.1 mL per fish, intraperitoneal injection) of LG21S strain to the artificial amberjack was 11% in the KS-7M strain immunized group, 0% in the LG13E strain immunized group, and 100% in the LG21S strain immunized group, as compared with 0% in the control group, showing a significantly higher survival rate in the LG21S strain immunized group than in the control group (p < 0.05, Fisher's exact test, one-sided test). Furthermore, as shown in FIG. 4, the survival rate 14 days after high-concentration challenge (8.0 x 10⁵ CFU/0.1 mL per fish, intraperitoneal injection) of LG21S strain to artificial amberjack was 0% in the KS-7M strain-immunized group, 0% in the LG13E strain-immunized group, and 100% in the LG21S strain-immunized group, as compared with 0% in the control group, showing a significantly higher survival rate in the LG21S strain immunized group than in the control group.

Thus, in the non-heated viable state, the inactivated antigens of new serotype/LG21S strain being negative for agglutination with both anti-L. garvieae type I serum (anti-KG- type serum and anti-KG+ type serum) and anti-L. garvieae type II serum in a viable state, but being positive for agglutination with anti-L. garvieae type I serum in heat-killed cells, showed high effective to the challenge of homo-type strain, whereas the inactivated antigens of L. garvieae type I and KG- type (KS-7M strain) and L. garvieae type II (LG13E strain) were unable to protect against the same attack. These results indicate that the LG21S strain is L. garvieae having new antigenicity that is different from both serotypes I and II, and further demonstrate that an inactivated vaccine preparation of "L. garvieae that is negative for agglutination with both anti-L. garvieae type I serum (anti-KG- type serum and anti-KG+ type serum) and anti-L. garvieae type II serum in a viable state, but positive for agglutination with anti-L. garvieae type I serum in heat-killed cells" is effective in preventing an α-streptococcosis caused by this bacterium.

### [Example 4]

In Example 4, the genetic homology between the eight strains of the new serotype isolated in Example 1 and existing strains was examined.

For the standard strains JCM10343 and KS-7M of L. garvieae, as well as the eight strains of the new serotype isolated in Example 1, 16S rRNA was sequenced, and the sequences were compared for the approximately 1,370 bp that could be decoded. As a result, all sequences for the parts that could be decoded were completely identical, and no mutations were found in the eight strains of the new serotype.

For the eight strains of the new serotype isolated in Example 1, the base sequence of the region from the glxR coding region to its vicinity was amplified by PCR using primers for distinguishing L. garvieae type I/II described in Non-Patent Document 4, and the PCR product was sequenced to obtain a decipherable 256 bp sequence. The base sequence of that region was completely identical for the eight strains. The obtained sequence is shown in SEQ ID NO:1.

Furthermore, as a comparative example, the PCR product of the KS-7M strain was sequenced in the same manner, and a decipherable sequence of 258 bp was obtained. The obtained sequence is shown in SEQ ID NO: 2. Note here that the obtained sequence of the KS-7M strain was completely identical to the base sequence of the region in the known standard strain Lg2 and the known ATCC49156.

Regarding the region amplified in this example, when the sequences of the KS-7M strain (see SEQ ID NO: 2), the known standard strain Lg2, and the known ATCC49156 were compared, the sequence of the new serotype (see SEQ ID NO: 1) showed mutations in three bases (a mutation from adenine to cytosine at position 62 in SEQ ID NO: 2, a deletion at position 119, and a deletion at position 143).

### [Example 5]

In Example 5, whether the inactivated vaccine prepared in Example 3 was effective also against the same infection in striped jack was examined.

As the test fish, 60 artificial striped jacks (delivered in February 2021, average body weight: 26 g) were prepared, and 30 of the fish were injected intraperitoneally with 0.1 mL of the inactivated vaccine prepared in Example 3 under anesthesia to form the LG21S strain immunization group, and the remaining 30 as the control group were injected intraperitoneally with 0.1 mL of PBS under the same conditions. After the injection, the fish were reared at 25°C for 14 days.

As in Example 3, the LG21S strain was selected as the challenge strain, and a bacterial liquid obtained by diluting the bacterial liquid 100-fold or 1,000-fold with PBS in the same manner as in Example 3, was prepared as a challenge bacterial liquid.

Fourteen days after immunization, the fish were challenged by intraperitoneally injecting 0.1 mL of a challenge solution per fish under anesthesia. After the challenge, the fish were reared in an aquarium tank at a set temperature of 25°C for 14 days, and the survival rates were compared.

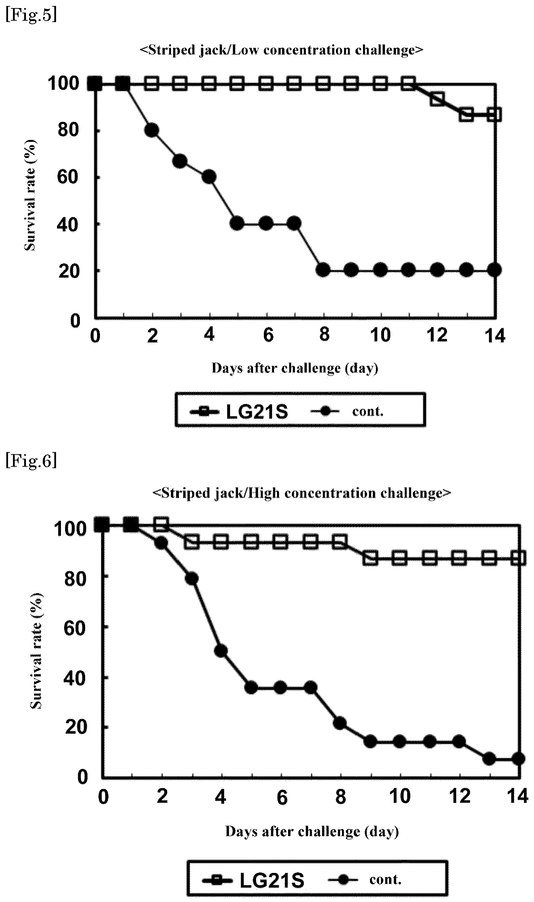

The results are shown in FIGs. 5 and 6. FIG. 5 is a graph showing the survival rate of artificial striped jack immunized with inactivated cells of the new serotype L. garvieae LG21S strain and then challenged with the LG21S strain at a low concentration (1.1 x 10⁵ CFU per fish), and FIG. 6 is a graph showing the survival rate of artificial striped jack immunized with inactivated cells of the new serotype L. garvieae LG21S strain and then challenged with the LG21S strain at a high concentration in (1.1 x 10⁴ CFU per fish). The horizontal axis of each graph (days after challenge) represents the days after the challenge, and the vertical axis (survival rate, unit: %) represents the survival rate after the challenge. In the graph, the line for "LG21S" represents the survival rate when immunization with inactivated cells of the new serotype L. garvieae LG21S strain was carried out, and the line for the "control group" represents the survival rate when PBS was administered as a control.

As shown in Figure 5, the survival rate 14 days after low-concentration challenge of artificial striped jack with the LG21S strain (1.1 x 10⁵ CFU per fish, intraperitoneal injection) was 87% in the immunized group, as compared with 20% in the control group showing a significant difference between the two groups (p < 0.05, Fisher's exact test, one-sided test). Similarly, as shown in FIG. 6, the survival rate 14 days after high-concentration challenge of artificial striped jack with the LG21S strain (1.1 x 10⁴ CFU per fish, intraperitoneal injection) was 87% in the immunized group, as compared with 7% in the control group, showing a significant difference between the two groups (p < 0.05, Fisher's exact test, one-sided test).

As described above, this example demonstrated that an inactivated vaccine preparation of "L. garvieae that is negative for agglutination with both anti-L. garvieae type I serum (anti-KG- type serum and anti-KG+ type serum) and anti-L. garvieae type II serum in a viable state, but positive for agglutination with anti-L. garvieae type I serum in heat-killed cells" is also effective in preventing α-streptococcosis in striped jack, which is caused by the same bacterium.

### [Example 6]

In Example 6, it was verified that that piscine streptococcosis caused by infection with the strain isolated in Example 1 is an infectious disease that cannot be prevented by existing vaccines.

Artificial yellowtail (delivered in June 2021, average body weight: 26 g) and artificial striped jack (delivered in June 2021, average body weight: 29 g) were immunized with the existing vaccine formulation "Picivac Note 4 (Picivac is a registered trademark)" according to the dosage and administration instructions (n = 18). As a positive control, the test antigen prepared using the strain isolated in Example 1 and the procedure described in Example 3 was administered instead, and as a negative control, PBS was administered instead.

As in Example 3, the LG21S strain was selected as the challenge strain and prepared as a challenge bacterial solution. After 14 days from the immunization, the fish were challenged with 0.1 mL of the challenge bacterial solution per fish (1.2 x 10⁸ CFU per fish) intraperitoneally under anesthesia. After the challenge, the fish were reared in an aquarium tank at a set water temperature of 25°C for 14 days, and the survival rates were compared.

As a result, while the survival rate was 0% in the negative control and 100% in the positive control, the survival rate in the group immunized with the existing vaccine formulation was 0%, the same as that in the negative control. These results showed that the novel piscine streptococcosis identified in Examples 1 and 2 was an infectious disease that cannot be prevented by existing vaccines.

### [Example 7]

In Example 7, whether the culture supernatant of the test strain in Example 3 also had immunogenicity or not was examined.

An inactivated bacterial solution (1.0 x 10⁹ CFU/mL) prepared in the same manner as in Example 3 was centrifuged at 12,000 G at 4°C for 10 minutes, and the isolated supernatant was allowed to pass through a 0.22 µm filter to completely remove the cells, which was used as the supernatant antigen. In addition, the cells remaining after removing the supernatant were suspended in PBS and centrifugally washed twice, and resuspended in PBS containing 0.2 vol% formalin, which was used as the bacterial antigen (1.0 x 10⁹ CFU/mL).

Artificial amberjack (delivered in June 2021, average body weight: 41 g) were immunized by intraperitoneal injection of 0.1 mL of supernatant antigen or bacterial antigen, and were reared at 25°C for 14 days after injection (n = 18). As a positive control, the inactivated bacterial liquid prepared in Example 3 was administered instead, and as a negative control, PBS was administered instead.

As in Example 3, the LG21S strain was selected as the challenge strain and prepared as a challenge bacterial solution. After 14 days from the immunization, the fish were challenged with 0.1 mL of the challenge bacterial solution per fish (9.7 x 10⁵ CFU per fish) intraperitoneally under anesthesia. After the challenge, the fish were reared in an aquarium tank at a set water temperature of 25°C for 14 days, and the survival rates were compared.

The results showed that while the survival rate was 0% in the negative control and 100% in the positive control, the survival rate when the cells antigen was administered was 100%, the same as that in the positive control, whereas when the supernatant antigen was administered, the survival rate was 0%, the same as that in the negative control. These results suggested that the immunogenicity of the culture supernatant was low in strains of the new serotype, and suggested that vaccine efficacy and production efficiency could be improved by using a concentrate of cell liquid made from cultured bacterial liquid.

### [Example 8]

In Example 8, causative bacteria isolated and identified in Examples 1 and 2 were attempted to isolate from yellowtail.

At first, the novel alpha-hemolytic streptococcosis shown in Example 1 was confirmed to cause damage mainly in amberjack and striped jack, but in August 2022, individuals suspected of similar α-hemolytic streptococcosis were discovered in a yellowtail aquaculture farm in Miyazaki Prefecture, Japan, and attempts were made to isolate and identify the causative bacterium.

The kidney, spleen, or brain of a yellowtail suspected of being infected in the aquaculture farm was punctured, and isolation and culture were attempted in the same manner as in Example 1, resulting in successful isolation of a strain derived from yellowtail. A hemolytic test of the isolated bacteria and a slide agglutination test in a viable state showed similar results to those of the LG21S and LG21M strains.

In addition, the results of biochemical property tests on the isolated bacteria were the same as those in Example 1 (Tables 3 and 4), and the results were negative to PyrA (L-pyroglutamic acid-β-naphthylamide, positive rate 74%) and βNAG (6-bromo-2-naphthyl-N-acetyl-β-D-glucosaminide, positive rate 10%), similar to the LG21S strain.

This yellowtail-derived isolate was cultured and prepared using the same procedures as in Example 2, then 10-, 100-, and 1,000-fold diluted with PBS to prepare test bacterial solutions. Under anesthesia, 0.1 mL of each of the bacterial solutions prepared at 10-fold concentration was injected intraperitoneally into artificial yellowtail, artificial amberjack, and artificial striped jack. After injection, the fish were reared at 25°C for 14 days, and the mortality rates were compared (n = 10). As a comparative example, an inactivated bacterial solution of the LG21S strain prepared in Example 3 was injected instead.

As a result, in artificial yellowtail, when the yellowtail-derived isolate was injected, the cumulative mortality rate 14 days after the injection was 50% at 1,000-fold dilution, 56% at 100-fold dilution, and 80% at 10-fold dilution, whereas when the inactivated bacterial solution of the LG21S strain was injected, the cumulative mortality rate was 56% at 1,000-fold dilution, 80% at 100-fold dilution, and 100% at 10-fold dilution. In artificial amberjack, when the yellowtail-derived isolate was injected, the cumulative mortality rate 14 days after injection was 100% at 1,000-fold dilution, 90% at 100-fold dilution, and 100% at 10-fold dilution, whereas when the inactivated bacterial solution of the LG21S strain was injected, the cumulative mortality rate was 100% at 1,000-fold dilution, 80% at 100-fold dilution, and 100% at 10-fold dilution. In artificial striped jacks, when the yellowtail-derived isolate was injected, the cumulative mortality rate 14 days after injection was 80% at 1,000-fold dilution, 70% at 100-fold dilution, and 80% at 10-fold dilution, whereas when an inactivated bacterial solution of the LG21S strain was injected, the cumulative mortality rate was 70% at 1,000-fold dilution, 80% at 100-fold dilution, and 60% at 10-fold dilution.

As mentioned above, this new α-hemolytic streptococcosis was found not only in amberjack and striped jack, but also in yellowtail, and the causative bacteria had the same properties and pathogenicity as the bacteria isolated from amberjack or striped jack. This suggests the possibility that this new α-hemolytic streptococcosis may be transmitted and spread in yellowtail aquaculture farms, just as it does in amberjack and striped jack. Meanwhile, in Example 3, the vaccine effect of the vaccine according to the present invention on yellowtail has already been demonstrated, and the present invention may be very useful in preventing this disease in yellowtail aquaculture farms.

### [Brief description of drawings]

FIG. 1 is a graph showing the survival rate of artificial yellowtail immunized with inactivated cells of the new serotype L. garvieae LG21S strain and then challenged with the LG21S strain at a low concentration in Example 3;
FIG. 2 is a graph showing the survival rate of artificial yellowtail immunized with inactivated cells of the new serotype L. garvieae LG21S strain and then challenged with the LG21S strain at a high concentration in Example 3;
FIG. 3 is a graph showing the survival rate of artificial amberjack immunized with inactivated cells of the new serotype L. garvieae LG21S strain and then challenged with the LG21S strain at a low concentration in Example 3;
FIG. 4 is a graph showing the survival rate of artificial amberjack immunized with inactivated cells of the new serotype L. garvieae LG21S strain and then challenged with the LG21S strain at a high concentration in Example 3;
FIG. 5 is a graph showing the survival rate of artificial striped jack immunized with inactivated cells of the new serotype L. garvieae LG21S strain and then challenged with the LG21S strain at a low concentration in Example 3; and
FIG. 6 is a graph showing the survival rate of artificial striped jack immunized with inactivated cells of the new serotype L. garvieae LG21S strain and then challenged with the LG21S strain at a high concentration in Example 3.

[Sequence Listing]

## Claims

1. An inactivated vaccine preparation against piscine streptococcosis caused by Lactococcus garvieae (scientific name "Lactococcus garvieae", the same hereinafter), comprising an inactivated cell of Lactococcus garvieae being negative for agglutination with anti-type I serum and anti-type II serum in a viable state.

2. The inactivated vaccine preparation according to claim 1, comprising an inactivated cell of Lactococcus garvieae being positive for agglutination with anti-type I serum of heat-killed cells.

3. The inactivated vaccine preparation according to claim 2, comprising an inactivated cell of Lactococcus garvieae comprising a sequence set forth in SEQ ID NO: 1 in a glxR coding region and in a vicinity of the region in genome.

4. A combined inactivated vaccine preparation comprising the inactivated vaccine according to claim 1, and an inactivated vaccine comprising inactivated cells of Lactococcus garvieae comprising a serotype I and/or serotype II.

5. A method for preventing piscine streptococcosis caused by Lactococcus garvieae, the method comprising administering the inactivated vaccine preparation according to claim 1.

6. A method for producing an inactivated vaccine preparation against piscine streptococcosis caused by Lactococcus garvieae, the method comprising inactivating Lactococcus garvieae cells being negative for agglutination with anti-type I serum and anti-type II serum in a viable state.

7. A strain of Lactococcus garvieae being negative for agglutination with anti-type I serum and anti-type II serum in a viable state.

8. A Lactococcus garvieae strain with international accession number NITE BP-03560 or NITE BP-03561.
